# EUROPEAN PATENT APPLICATION

(11) **EP 3 939 589 A1**
(43) Date of publication of application: **19.01.2022**
(21) Application number: 20185893.3
(22) Date of filing: 15.07.2020
(51) Int. Cl.: A61K 31/4439, A61K 9/20, A61P 7/06, A61K 9/16

(54) **HIGH DRUG LOAD TABLET COMPRISING VOXELOTOR**

(71) Applicant: SANDOZ AG, 4056 Basel (CH)
(72) Inventor: MARTIN, Nolwenn, 6250 Kundl (AT); KREKELER, Andreas, 83043 Bad Aibling (DE); GUGGENBERGER, Guenther, 6230 Brixlegg (AT)
(74) Representative: Lederer & Keller Patentanwälte Partnerschaft mbB

(57) **Abstract**

A tablet comprising at least 60% w/w of voxelotor and at least 10% w/w of low-substituted hydroxypropyl cellulose. Process for the preparation of a tablet comprising the process steps of blending voxelotor with low-substituted hydroxypropyl cellulose and, optionally, further excipients and compressing the resulting blend into a tablet. The tablet according for use in the treatment of haematological disorders such as sickle cell disease.

## Description

### TECHNICAL FIELD

The present invention relates to a high drug load pharmaceutical tablet containing the active ingredient voxelotor. The invention also relates to a process for preparing such a tablet and to its use for the treatment of haematological disorders such as sickle cell disease.

### BACKGROUND ART

A particularly difficult problem facing the pharmaceutical and medical communities is patient compliance with dosing regimens. Lack of compliance can result in lower levels of drug in the target tissue and the disease may "escape" the effects of the drug since it is not present at inhibitory concentrations. The pharmaceutical/medical community has focused drug development clinical trials on simple dosing regimens to promote compliance. One particular problem for creating acceptable dosing regimens is when large amounts of a drug need to be delivered to a patient: there is a limit to the size of a tablet a patient is able to swallow and the more tablets a patient has to take the more likely it is that they will make a mistake, resulting in non-compliance. There is thus a need for formulations useful for the delivery of large amounts of drug, with acceptable size and manageable pill burden.

Formulation of pharmaceutical tablets typically involves mixing the active pharmaceutical ingredient (API; the drug) with one or more inactive ingredients (i.e., excipients). Tablets that contain low doses (e.g., less than 50 mg drug per dose) will often be formulated with more excipient on a weight basis than the API to facilitate the manufacturing process (e.g., compaction), yet still result in small tablets that are easy for the patient to swallow. Since the excipients make up a substantial portion of the total tablet weight, the processing and manufacturability of the tablets are readily adjusted regardless of the properties of the drug agent.

Conversely, with high dose drugs, the characteristics of the tablet are strongly influenced by the properties of the API. If these properties are not compatible with commercial manufacturing requirements, the formulator is faced with producing larger tablets (adding excipients to solve the manufacturing problems), or dividing the desired amount of active ingredient in multiple tablets. Both alternatives negatively impact patient compliance.

Voxelotor is a small molecule active pharmaceutical ingredient indicated for the treatment of sickle cell disease, a disorder of red blood cells. The chemical name of voxelotor is 2-hydroxy-6-((2-(1-isopropyl-1*H*-pyrazol-5-yl)pyridin-3-yl)methoxy)benzaldehyde. The recommended authorised dosage is 1,500 mg once a day. Voxelotor is currently sold by Global Blood Therapeutics as 500 mg tablets under the brand name Oxbryta. This means that three tablets must be taken each day by the patient in order to reach the recommended daily dose.

WO 2015/120133 A1 discloses the chemical synthesis of voxelotor. It also discloses the preparation, among others, of two anhydrous crystalline forms, denominated form I and form II. WO 2015/120133 A1 reports in paragraphs [0186-187] that form I is not physically stable at 40°C / 75% RH and converts into form II.

WO 2018/071678 A1 discloses high drug load tablets comprising from 50 to 70% voxelotor and high compactable microcrystalline cellulose as a filler. WO 2018/071678 A1 reports in paragraph [0098] and in paragraph [0238] that the bulk density of form II of voxelotor is low and its flowability poor. Upon milling, the flowability decreases even more. The compression characteristics of form II of voxelotor are also poor, exhibiting low tensile strength which does not increase significantly with compression force. Form II of voxelotor is also prone to sticking to steel surfaces, such as in processing equipment for tablet manufacture. Overall, form II of voxelotor compression profile is not acceptable and requires substantial formulation efforts to overcome its poor compression properties.

In the examples of WO 2018/071678 A1, tablets containing 60% of voxelotor were prepared by dry granulation using a roller compactor. As excipients, microcrystalline cellulose was used as filler, croscarmellose sodium as disintegrant, magnesium stearate as lubricant; each of these excipients was used both as intragranular and extragranular component.

There still exists a need for formulations containing voxelotor that have a high drug load and therefore a small size and low weight. Such formulations should be able to incorporate higher drug loads than the prior art, minimum amounts of excipients and as few different excipients as possible. Their manufacturing process should be simple and efficient. It is also required that the formulations dissolve fast in order to provide a suitable bioavailability. Formulations in the form of a tablet should have good tensile strength and low friability.

Finally, voxelotor formulated in such formulations should be chemically and physically stable.

### BRIEF DESCRIPTION OF THE INVENTION

In order to solve this problem, the present inventors have carried out significant studies concerning suitable excipients and preparation methods for preparing formulations containing voxelotor. It has surprisingly been found that blends comprising voxelotor and the disintegrant low-substituted hydroxypropyl cellulose (L-HPC) can be easily compressed into tablets having a high drug load. Said tablets have thus a small size and low weight, exhibit fast dissolution, have good tensile strength and low friability.

The tablets of the present invention are suited for oral administration. Advantageously, they can be prepared by direct compression, i.e. without the need of dry or wet granulation. Moreover, it has surprisingly been found that L-HPC has a stabilising effect on voxelotor, which is physically and chemically stable when incorporated into the tablets of the present invention.

### Brief description of the drawings

Figure 1 depicts the dissolution profile of the tablet of example 1.
Figure 2 depicts the dissolution profile of the tablets of example 6, i.e. tablets prepared with coarse particles (API d(90) / d(50) / d(10) of 157 / 62 / 21 µm; symbols: ■) and tablets prepared with fine particles (API d(90) / d(50) / d(10) of 54 / 22 / 7 µm; symbols: ●). As a comparison, dissolution profile of a 100 mg reference tablet made according to WO 2018/071678 A1 (tablet G2, example 2, table 2 and figure 1 of WO 2018/071678 A1; symbols: ▲ and dotted line) is also represented.

### Definitions

All percentages in the present specification are on a weight basis and refer to the total weight of the tablet. It is to be understood that a value of e.g. 60% includes all values from 59.50% to 60.49%. The same applies to all other percentages disclosed herein.

Crystalline form I of voxelotor is characterized by at least two, three, or four X-ray powder diffraction peaks (Cu Ka radiation) selected from 12.82°, 15.74°, 16.03°, 16.63°, 17.60°, 25.14°, 25.82° and 26.44° 2θ (each ±0.2°2θ).

Crystalline form II of voxelotor is characterized by at least two, three, or four X-ray powder diffraction peaks (Cu Ka radiation) selected from 13.37°, 14.37°, 19.95° and 23.92° 2θ (each ±0.2°2θ).

### DETAILED DESCRIPTION OF THE INVENTION

The present invention relates to a tablet comprising at least 60% w/w of voxelotor and at least 10% w/w of low-substituted hydroxypropyl cellulose. Said tablet may comprise at least 65% w/w of voxelotor, preferably at least 70% w/w of voxelotor, more preferably 75% w/w of voxelotor, even more preferably at least 80% w/w of voxelotor. Alternatively, the tablet according of the present invention comprises from 50% to 90% w/w of voxelotor, preferably from 55% to 85% w/w of voxelotor, even more preferably from 60% to 80% w/w of voxelotor.

In a preferred embodiment, the tablet according of the present invention comprises at least 15% w/w of low-substituted hydroxypropyl cellulose, preferably at least 20% w/w of low-substituted hydroxypropyl cellulose. Alternatively, the tablet according of the present invention comprises from 10% to 40% w/w of low-substituted hydroxypropyl cellulose, preferably from 15% to 30% w/w of low-substituted hydroxypropyl cellulose. The low-substituted hydroxypropyl cellulose used in the tablets of the present invention can be of any grade. A particularly preferred grade of low-substituted hydroxypropyl cellulose is type LH-21.

In a particularly preferred embodiment, the tablet of the present invention does not comprise microcrystalline cellulose. The presence of such filler is not required to obtain a tablet according to the present invention having good compression properties. For this reason, higher drug load can be achieved than with the tablets according to the prior art.

As it will be explained below, direct compression is a particularly advantageous process for the preparation of the tablets of the present invention. The skilled person is able to determine by analysis whether or not a tablet has been made via direct compression or another preparation method, such as dry or wet granulation. Therefore, particularly preferred tablets according to the present invention are tablets obtained by direct compression.

In one embodiment, the tablet of the present invention contains 100 mg, 300 mg, 500 mg, 750 mg, 1000 mg or 1500 mg of voxelotor, calculated on voxelotor free form. Preferably, it contains 500 mg of voxelotor.

Voxelotor can be present in the tablet of the invention in free form (free base) or as an acid or base addition salt. Alternatively, a co-crystal of voxelotor can be used instead. In particularly preferred embodiments, either the free form of voxelotor or a co-crystal with 2,5-dihydroxybenzoic acid in a voxelotor / 2,5-dihydroxybenzoic acid ratio of 2:1 are employed.

Voxelotor can be present in amorphous form or in crystalline form. In a preferred embodiment, crystalline voxelotor is employed, preferably crystalline form II of voxelotor. However, the crystalline form of the voxelotor for use in the present invention is not particularly limited and other crystalline forms can also be used.

In order to ensure a rapid dissolution of the tablet, voxelotor should be present in the form of fine particles. Such particles can be obtained by standard techniques well-known in the art, such as milling, crushing or sieving. In a preferred embodiment, the particle size distribution of voxelotor has a d(90) of less than 100 µm, preferably of less than 80 µm, more preferably of less than 60 µm when measured by laser diffraction.

The tablet of the present invention may contain more than one active ingredient. That is, besides voxelotor another active ingredient may be present. In a preferred embodiment the tablet may only contain one pharmaceutically active ingredient, namely voxelotor.

The tablets according to the present invention may contain one or more further excipients, preferably selected from the group consisting of fillers, disintegrants, binders, surfactants, lubricants and glidants.

In preferred embodiments, the tablets include one or more surfactants (also known as wetting agents), e.g. an ionic surfactant or a non-ionic surfactant. Surfactants are normally selected to maintain the drug or drugs in close association with water, a condition that is believed to improve bioavailability of the composition. Ionic surfactants can be anionic surfactants, such as sodium dodecyl sulfate (SDS), or cationic surfactants, such as cetyl pyridinium chloride and cetyltriethylammonium bromide. Non-ionic surfactants are, e.g., polysorbates, which are sold under the tradenames span, tween and poloxamer. In a particularly preferred embodiment, the surfactant is sodium dodecyl sulphate, preferably in an amount between 0.5% w/w and 3% w/w, more preferably in an amount of 1.5% w/w.

In preferred embodiments, the tablets of the present invention include one or more lubricants. Lubricants can be regarded as substances which are suitable to reduce friction, such as static friction, sliding friction and rolling friction. In particular, lubricants reduce the shearing forces which can occur on the borderline between tablet and mould, especially the sliding friction found during tablet pressing between the punch moving up and down in the die and the die wall on the one hand and between the edge of the tablet and the die wall on the other hand. Lubricants include glyceryl derivatives, oils, sodium or magnesium stearyl fumarate, stearic acid, calcium or magnesium stearate, talc, etc. Lubricants include for example magnesium stearate, calcium stearate, glyceryl monostearate, glyceryl palmitostearate, hydrogenated castor oil, hydrogenated vegetable oil, mineral oil, polyethylene glycol, sodium benzoate, sodium stearyl fumarate, stearic acid, talc and zinc stearate. In a particularly preferred embodiment, the lubricant is magnesium stearate.

In one embodiment, the tablets of the present invention include one or more fillers. Fillers increase the bulk of a solid pharmaceutical composition. Fillers include cellulose derivatives, sugar derivatives, starch derivatives, calcium phosphate, oxides / carbonates / sulfates / chlorides of magnesium or calcium or sodium. Fillers include, for example, microcrystalline cellulose, microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, lactose monohydrate, magnesium carbonate, magnesium oxide, maltodextrin, mannitol, polymethacrylates, potassium chloride, powdered cellulose, spray dried lactose, sodium chloride, sorbitol and talc.

In one embodiment, the tablets of the present invention include one or more further disintegrants, in addition to L-HPC. Disintegrants include cellulose derivatives, hydrophilic polymers, calcium phosphate, alginic acid, colloidal silicon dioxide, starch, sodium starch glycolate, aluminium silicates, guar gum etc. Disintegrants include, for example, alginic acid, carboxymethylcellulose calcium, carboxymethylcellulose sodium, colloidal silicon dioxide, croscarmellose sodium, crospovidone, guar gum, magnesium aluminum silicate, methyl cellulose, microcrystalline cellulose, polyvinyl polypyrrolidone, powdered cellulose, pregelatinized starch, sodium alginate, sodium starch glycolate and starch.

In one embodiment, the tablets of the present invention include one or more binders. Binders help bind the active ingredient and other excipients together after compression. Binders include cellulose derivatives, sugar derivatives, starch derivatives, gelatine, guar gum, magnesium aluminium silicate, sodium alginate, stearic acid, hydrophilic or hydrophobic polymers. Binders for solid pharmaceutical compositions include for example acacia, alginic acid, carbomer, carboxymethylcellulose sodium, copovidone, dextrin, ethyl cellulose, gelatin, guar gum, hydrogenated vegetable oil, hydroxyethyl cellulose, hydroxypropyl cellulose, hydroxypropyl methyl cellulose, liquid glucose, magnesium aluminum silicate, maltodextrin, methylcellulose, polymethacrylates, polyvinyl pyrrolidone, pregelatinized starch, sodium alginate and starch.

In one embodiment, the tablets of the present invention include one or more glidants. Glidants can be added to improve the flowability of non-compacted solid composition and improve the accuracy of dosing. Excipients that can function as glidants include for example colloidal silicon dioxide, magnesium trisilicate, powdered cellulose, starch, talc and tribasic calcium phosphate.

In a preferred embodiment, the tablet of the present invention consists of voxelotor, low-substituted hydroxypropyl cellulose, sodium dodecyl sulphate, magnesium stearate and, optionally, a glidant preferably selected from the group consisting of colloidal silicon dioxide, tribasic calcium phosphate and a mixture thereof.

In a particularly preferred embodiment, the tablet of the present invention consists of voxelotor, low-substituted hydroxypropyl cellulose, sodium dodecyl sulphate and magnesium stearate.

Various tablet formulations may be made in accordance with the present invention. These include tablet dosage forms such as sugar-coated tablets, film-coated tablets, enteric-coated tablets, multiple-compressed tablets, prolonged action tablets and the like. The tablet of the present invention can be coated or uncoated. The skilled person is familiar with suitable coating compositions and processes.

Sugar-coated tablets (SCT) are compressed tablets containing a sugar coating. Such coatings may be coloured and are beneficial in covering up drug substances possessing objectionable tastes or odours and in protecting materials sensitive to oxidation. Film-coated tablets (FCT) are compressed tablets that are covered with a thin layer or film of a watersoluble material. A number of polymeric substances with film-forming properties may be used. The film coating imparts the same general characteristics as sugar coating with the added advantage of a greatly reduced time period required for the coating operation. Enteric-coated tablets are also suitable for use in the present invention. Enteric-coated tablets (ECT) are compressed tablets coated with substances that resist dissolution in gastric fluid but disintegrate in the intestine. Enteric coating can be used for tablets containing drug substances that are inactivated or destroyed in the stomach, for those which irritate the mucosa or as a means of delayed release of the medication.

Processes for preparing the tablets according to the invention are known in the art. Voxelotor, low-substituted hydroxypropyl cellulose and, optionally, further excipients are blended together and then compressed into a tablet, e.g. with an appropriate press and press force. Ingredients can be optionally sieved or milled before blending to reduce their particle size. The tablet may be optionally coated. In a preferred embodiment, the tablets according to the present invention are prepared by direct compression, as shown in the examples. Direct compression produces a more uniform tablet without granules. Alternatively, the tablets are prepared by dry or wet granulation.

The tablets according to the present invention are preferably for use in the treatment of haematological disorders such as sickle cell disease.

### Advantages

The tablets according to the present invention allow for a higher drug load than those of the prior art, which exemplifies only tablets with a drug load of 60%. This allows the preparation of smaller and lighter tablets that can be more easily swallowed. Moreover, a higher drug load allows for higher dosages in a single tablet, such as 666, 750 or 1000 mg. This reduces the pill burden for the patients and thus increases patient compliance.

Unlike the tablets of the prior art, which are manufactured by dry granulation, the tablets of the present invention can be easily manufactured by direct compression. This is a very simple process that involves no extra steps and thus reduces production costs.

Another advantage of the present invention is the use of a limited amounts of excipients, as only low-substituted hydroxypropyl cellulose is needed to obtain a high drug load tablet containing voxelotor having low friability and good tensile strength. This dispenses with the use of a filler. Tablets of the prior art, instead, make use of a filler, microcrystalline cellulose, and a disintegrant, croscarmellose sodium. This simplification in the ingredients used allows to further streamline the production process and further reduce costs.

Thanks to the higher drug load of the tablets of the present invention, incorporation of salts and co-crystals of voxelotor is possible. Such salts and co-crystal add bulk to the tablet compared to voxelotor free base. Nevertheless, in the formulation of the present invention, the use of such salts or co-crystals is possible while at the same time ensuring a small size and weight of the tablet.

Finally, L-HPC has a stabilising effect on voxelotor, which is physically and chemically stable when incorporated into the tablets of the present invention. More particularly, the tablet of the present invention can stabilise voxelotor form I, which is thus physically and chemically stable and does not convert to form II. This allows a wider choice of crystal forms for the API to be used in the tablet.

The following examples are illustrative without restricting the scope of protection. If in the examples a process detail is not explicitly described, a skilled person can easily find such detail according to the general practice in the art.

### Analytical methods

Tablet hardness: tablet hardness or tablet breaking force is the measure of the mechanical integrity of tablets. Tablet hardness was measured using a standard hardness tester as described in the United States Pharmacopoeia ("USP").

Tablet friability: tablet friability is a test of the ability of tablets to withstand mechanical stresses that determines their resistance to chipping and surface abrasion by tumbling them in a rotating cylinder. The percentage weight loss after tumbling is referred to as the friability of the tablets. Tablet friability for the tablets of the invention was tested using a friability tester designed as per USP specifications for 4 minutes (rotation speed: 25 rpm).

### Tablet dissolution:

The tablet dissolution was tested in an Agilent dissolution apparatus 708-DS:

| | |
|---|---|
| Method | USP, Type II - Paddles |
| Sample | tablets |
| Dissolution medium 900 ml 0.1 N HCl + 0.5% Tween 80 | |
| Temperature | 37°C ± 0.5°C |
| Agitation | paddle, 75 rpm |
| Detector | UV |
| Wave length | 345 nm |

### Particle size distribution:

Particle size distribution was determined by laser diffraction using a MALVERN Mastersizer 2000 with a Hydro 2000S (liquid dispersion unit).

### Procedure:

The cell was filled with water and degassed. Solid sample (voxelotor) was then added to the dispersion unit, followed by addition of 3-5 drops of 5% Tween20 solution until obscuration between 10-30% was reached. The powder was dispersed for 4 min at a pump speed of 2500 rpm, before particle size was measured (3x).

### Instrument settings:

| | |
|---|---|
| Size range: | 0.02-2000 µm |
| Dispersant: | water |
| Dispersant RI: | 1.330 |
| Calculation: | Fraunhofer |
| Delay period: | 4 min |
| Pump speed: | 2500 rpm |
| Obscuration limits: | 10-30% |
| Analysis model: | general purpose |
| Sensitivity: | normal |
| Particle shape: | irregular |
| Background time: | 12 sec |
| Sample time: | 10 sec |
| Volume distribution result: d(0.1); d(0.5); d(0.9) in µm | |

### Powder X-ray diffraction

The crystalline form of voxelotor according to the present invention was investigated by powder X-ray diffraction, which was performed with a PANalytical X'Pert PRO diffractometer equipped with a theta/theta coupled goniometer in transmission geometry, Cu-Kalpha1,2 radiation (wavelength 0.15419 nm) with a focusing mirror and a solid state PIXcel detector. The sample was gently ground in an agate mortar with a pestle before the measurement. The diffractogram was recorded at a tube voltage of 45 kV and a tube current of 40 mA, applying a stepsize of 0.013° 2-theta with 40s per step (255 channels) in the angular range of 2° to 40° 2-Theta at ambient conditions. A typical precision of the 2-Theta values is in the range of ± 0.2° 2-Theta, preferably of ± 0.1° 2-Theta.

### Example 1: tablet formulation with 80% drug load manufactured by direct compression

| **Component** | **Amount per tablet (mg)** | **% w/w** |
|---|---|---|
| Voxelotor form II | 666 | 80.0 |
| L-HPC (type LH-21) | 136 | 16.3 |
| Sodium dodecyl sulphate | 13 | 1.5 |
| Magnesium stearate | 18 | 2.2 |
| **TOTAL** | **833** | **100.0** |

Voxelotor, L-HPC and SDS were separately sieved (500 µm) before dispensing. The dispensed materials were passed twice through a 355 µm sieve and dry mixed for 12 minutes. Magnesium stearate was added to the mixture, which was mixed again for 2 minutes. The blend was compressed into tablets using the following parameters:

**Tabletting machine: Röltgen FlexiTab S**

| | |
|---|---|
| Tablet size: | 9 mm x 19 mm |
| Tablet shape: | oblong |
| Compression force: | 12.7 kN |

Friability and breaking force were measured with the following results:

| | |
|---|---|
| Friability: | 0.47% |
| Breaking force: | 109 N |

### Example 2: tablet formulation with 60% drug load manufactured by direct compression or wet granulation

| **Component** | **Amount per tablet (mg)** | **% w/w** |
|---|---|---|
| Voxelotor form II | 500 | 60.0 |
| L-HPC (type LH-21) | 302 | 36.3 |
| Sodium dodecyl sulphate | 13 | 1.5 |
| Magnesium stearate | 18 | 2.2 |
| **TOTAL** | **833** | **100.0** |

### Direct compression

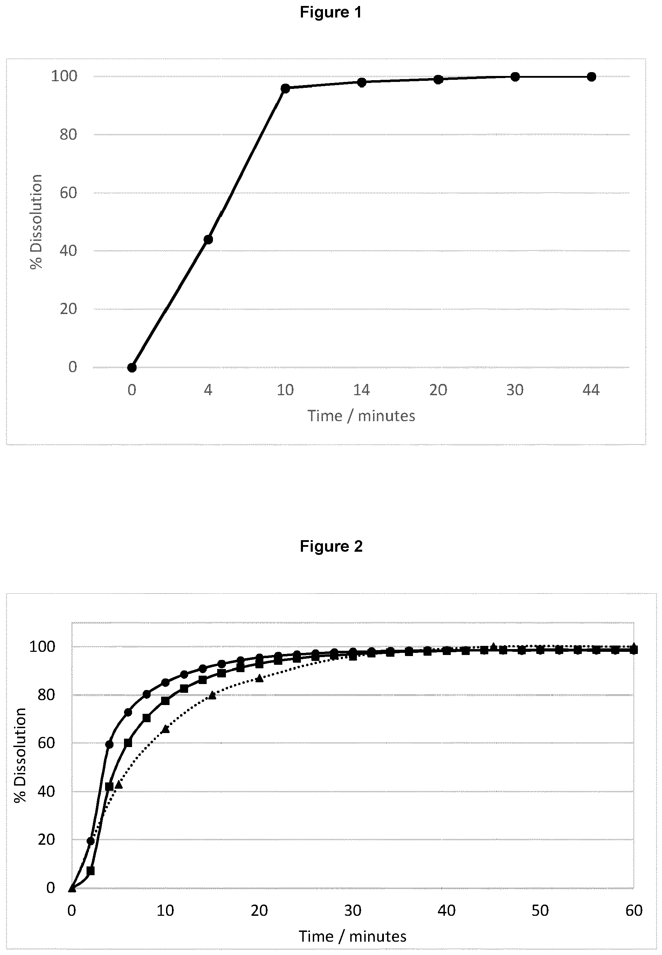

The same manufacturing process as the one reported in example 1 was employed. The dissolution profile is reported in figure 1. Friability and breaking force were measured under the conditions reported in example 1 obtaining the following results:

| | |
|---|---|
| Friability: | 0.02% |
| Breaking force: | 118 N |

### Wet granulation

A mixture of Voxelotor and L-HPC was granulated with water (60 g water per 100 g dry material). Granules were dried at 40-50°C for 5h. Granules were sieved through a 355 µm sieve. SDS was added to the granules and mixed for 5 minutes. Magnesium stearate was added to the mixture, which was mixed again for 2 minutes.

The blend was compressed into tablets under the conditions reported in example 1. Friability and breaking force were measured under the conditions reported in example 1 obtaining the following results:

| | |
|---|---|
| Friability: | 0.35% |
| Breaking force: | 178 N |

Both preparations were repeated with similar results using crystalline form I of voxelotor instead of crystalline form II.

### Example 3: tablet formulation with 60% drug load manufactured by direct compression or wet granulation

| **Component** | **Amount per tablet (mg)** | **% w/w** |
|---|---|---|
| Voxelotor form II | 500.0 | 58.8 |
| L-HPC (type LH-21) | 302.1 | 35.5 |
| Sodium dodecyl sulphate | 12.5 | 1.5 |
| Magnesium stearate | 18.7 | 2.2 |
| Colloidal silicon dioxide | 8.3 | 1.0 |
| Tribasic calcium phosphate | 8.3 | 1.0 |
| **TOTAL** | **849.9** | **100.0** |

### Direct compression

The same manufacturing process as the one reported in example 1 was employed.

### Wet granulation

The same manufacturing process as the one reported in example 2 was employed.

### Example 4: tablet formulations with 60% drug load (based on voxelotor free base) manufactured by direct compression or wet granulation

A)

| **Component** | **Amount per tablet (mg)** | **% w/w** |
|---|---|---|
| Voxelotor cocrystal with 2,5-dihydroxybenzoic acid (ratio 2:1) | 614 | 73.7 |
| L-HPC (type LH-21) | 188 | 22.6 |
| Sodium dodecyl sulphate | 13 | 1.5 |
| Magnesium stearate | 18 | 2.2 |
| **TOTAL** | **833** | **100.0** |

B)

| **Component** | **Amount per tablet (mg)** | **% w/w** |
|---|---|---|
| Voxelotor cocrystal with 2,5-dihydroxybenzoic acid (ratio 2:1) | 614.2 | 72.3 |
| L-HPC (type LH-21) | 187.9 | 22.0 |
| Sodium dodecyl sulphate | 12.5 | 1.5 |
| Magnesium stearate | 18.7 | 2.2 |
| Colloidal silicon dioxide | 8.3 | 1.0 |
| Tribasic calcium phosphate | 8.3 | 1.0 |
| **TOTAL** | **849.9** | **100.0** |

### Direct compression

The same manufacturing process as the one reported in example 1 was employed.

### Wet granulation

The same manufacturing process as the one reported in example 2 was employed.

### Example 5: stability data

The physical and chemical stabilities of the tablets of example 3 made by direct compression (DC) and wet granulation (WG) were evaluated after 1 month at 40°C / 75% RH in open and closed HDPE bottles without desiccant. X-ray diffraction (XRD) was used to determine the crystalline form. Moreover, the voxelotor release after 15 min was measured. The results are reported in the following table (T0 = time 0, T1 = after 1 month):

| Manufacturing procedure | DC | | WG | | DC | | WG |
|---|---|---|---|---|---|---|---|
| Storage conditions: | open | closed | open | closed | open | closed | open |
| Form (XRD) T0 | II | II | II | II | I | I | I |
| Form (XRD) T1 | II | II | II | II | I | I | I |
| Total impurities, T0 (%) | 0.22 | 0.22 | 0.22 | 0.22 | 0.54 | 0.54 | 0.50 |
| *(Main impurity T0, %)* | *(0.17)* | *(0.17)* | *(0.17)* | *(0.17)* | *(0.38)* | *(0.38)* | *(0.35)* |
| Total impurities, T1 (%) | 0.38 | 0.40 | 0.41 | 0.39 | 0.68 | 0.64 | 0.67 |
| *(Main impurity T1, %)* | *(0.21)* | *(0.20)* | *(0.20)* | *(0.19)* | *(0.40)* | *(0.36)* | *(0.38)* |
| Total impurity increase, T1 %) | 0.16 | 0.18 | 0.19 | 0.17 | 0.14 | 0.10 | 0.17 |
| *(Main impurity increase T1, %*) | *(0.04)* | *(0.03)* | *(0.03)* | *(0.02)* | *(0.02)* | *(0.02)* | *(0.03)* |
| Dissolution T0 | 95% | 95% | 93% | 93% | 100% | 100% | 100% |
| Dissolution T1 | 92% | 94% | 92% | 93% | 98% | 100% | 100% |

As it can be seen, all tested tablets were physically and chemically stable in all conditions and exhibited good dissolution. Surprisingly, tablets made with voxelotor crystal form I showed no conversion to crystal form II.

### Example 6: particle size distribution

The effects of particle size distribution on tablet dissolution was tested by using two API lots, one with coarse particles, with d(90) / d(50) / d(10) of 157 / 62 / 21 µm, and one with fine particles, with d(90) / d(50) / d(10) of 54 / 22 / 7 µm. 100 mg tablets were prepared according to the procedure of example 2 (direct compression). The results are reported in figure 2, which also includes a comparison with 100 mg tablets made according to WO 2018/071678 A1 (tablet G2, example 2, table 2 and figure 1). As it can be seen, both tablet formulations of the present invention exhibit rapid and complete dissolution. The dissolution of tablets made with fine voxelotor was quicker than that of tablets made with coarse voxelotor. Both tablets according to the present invention exhibited more rapid dissolution than the reference tablet of the prior art.

## Claims

1. A tablet comprising at least 60% w/w of voxelotor and at least 10% w/w of low-substituted hydroxypropyl cellulose.

2. The tablet according to claim 1 comprising at least 65% w/w of voxelotor, preferably at least 70% w/w of voxelotor, more preferably 75% w/w of voxelotor, even more preferably at least 80% w/w of voxelotor.

3. The tablet according to claims 1 to 2 comprising at least 15% w/w of low-substituted hydroxypropyl cellulose, preferably at least 20% w/w of low-substituted hydroxypropyl cellulose.

4. The tablet according to any of the preceding claims, wherein said tablet does not comprise microcrystalline cellulose.

5. The tablet according to any of the preceding claims, wherein the tablet is obtained by direct compression.

6. The tablet according to any of the preceding claims, wherein voxelotor is present as an acid or base addition salt or as a co-crystal, preferably as a co-crystal with 2,5-dihydroxybenzoic acid in a voxelotor / 2,5-dihydroxybenzoic acid ratio of 2:1.

7. The tablet according to any of the preceding claims, wherein the tablet contains 500 mg voxelotor, calculated on voxelotor free form.

8. The tablet according to any of the preceding claims, wherein the particle size distribution of voxelotor has a d(90) of less than 100 µm, preferably of less than 80 µm, more preferably of less than 60 µm when measured by laser diffraction.

9. The tablet according to any of the preceding claims, wherein voxelotor is present in crystalline form, preferably having an X-ray powder diffraction pattern of form II **characterized by** at least two, three, or four X-ray powder diffraction peaks (Cu Ka radiation) selected from 13.37°, 14.37°, 19.95° and 23.92° 2θ (each ± 0.2°2θ) or an X-ray powder diffraction pattern of form I **characterized by** at least two, three, or four X-ray powder diffraction peaks (Cu Ka radiation) selected from 12.82°, 15.74°, 16.03°, 16.63°, 17.60°, 25.14°, 25.82° and 26.44° 2θ (each ±0.2°2θ).

10. The tablet according to any of the preceding claims comprising one or more further excipients, preferably selected from the group consisting of fillers, disintegrants, binders, surfactants, lubricants and glidants.

11. The tablet according to any of the preceding claims comprising a surfactant, preferably sodium dodecyl sulphate, and a lubricant, preferably, magnesium stearate.

12. The tablet according to claim 10 consisting of voxelotor, low-substituted hydroxypropyl cellulose, sodium dodecyl sulphate, magnesium stearate and, optionally, a glidant.

13. Process for the preparation of a tablet according to claims 1 to 12 comprising the process steps of blending voxelotor with low-substituted hydroxypropyl cellulose and, optionally, further excipients and compressing the resulting blend into a tablet.

14. A process according to claim 13, wherein the blended powders are directly compressed into a tablet.

15. The tablet according to any of claims 1 to 12 for use in the treatment of haematological disorders such as sickle cell disease.
